(19) Europäisches Patentamt
European Patent Office
Office européen des brevets

(11) **EP 3 987 280 B1**

(12) **FASCICULE DE BREVET EUROPEEN**

(45) Date de publication et mention
de la délivrance du brevet:
**01.01.2025 Bulletin 2025/01**

(21) Numéro de dépôt: **19806242.4**

(22) Date de dépôt: **22.11.2019**

(51) Classification Internationale des Brevets (IPC):
**G01N 27/12** *(2006.01)*

(52) Classification Coopérative des Brevets (CPC):
**G01N 27/123**

(86) Numéro de dépôt international:
**PCT/EP2019/082209**

(87) Numéro de publication internationale:
**WO 2020/109160 (04.06.2020 Gazette 2020/23)**

(54) **PROCEDE DE DETECTION D'AU MOINS UNE QUANTITE DE GAZ D'AU MOINS UN GAZ PREDETERMINE A PARTIR D'UN CAPTEUR DE MESURE D'UNE PLURALITE DE GAZ**

VERFAHREN ZUR DETEKTION MINDESTENS EINER GASMENGE MINDESTENS EINES VORBESTIMMTEN GASES DURCH EINEN MESSFÜHLER EINER MEHRZAHL VON GASEN

METHOD FOR DETECTING AT LEAST ONE GAS QUANTITY OF AT LEAST ONE PREDETERMINED GAS BY A MEASUREMENT SENSOR OF A PLURALITY OF GASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2018 FR 1871971**

(43) Date de publication de la demande:
**27.04.2022 Bulletin 2022/17**

(73) Titulaire: **ELLONA**
**31400 Toulouse (FR)**

(72) Inventeurs:
• **BEN HAMOUDA, Franck**
**31500 Toulouse (FR)**
• **MIFSUD, Jean-Christophe**
**82400 Goudourville (FR)**

(74) Mandataire: **Argyma**
**14 Boulevard de Strasbourg**
**31000 Toulouse (FR)**

(56) Documents cités:
**WO-A1-2006/057550     DE-A1- 19 639 072**
**US-A- 5 889 198**

• **KOHLER H ET AL: "New applications of tin oxide gas sensors - I. Molecular identification by cyclic variation of the working temperature and numerical analysis of the signals", SENSORS AND ACTUATORS B: CHEMICAL, ELSEVIER BV, NL, vol. 61, no. 1-3, 14 December 1999 (1999-12-14), pages 163 - 169, XP004185148, ISSN: 0925-4005, DOI: 10.1016/S0925-4005(99)00286-5**

## Description

### DOMAINE TECHNIQUE ET OBJET DE L'INVENTION

La présente invention concerne le domaine des capteurs de gaz et vise plus particulièrement un procédé de commande d'un capteur de gaz.

### ETAT DE LA TECHNIQUE

Aujourd'hui, il est courant d'utiliser un capteur pour mesurer différents gaz, notamment dans l'haleine d'une personne afin de mesurer le taux d'alcoolémie, à l'intérieur d'un bâtiment afin de déterminer la qualité de l'air, etc.

Pour réaliser de telles mesures, il est connu un capteur oxydo-métallique, également désigné capteur MOX, qui présente un coût et un encombrement limités. Un tel capteur MOX comprend une couche sensible dont la conductivité varie en présence de certains gaz et une couche chauffante sur laquelle est montée la couche sensible. La couche chauffante est alimentée en énergie électrique par une tension continue afin de chauffer la couche sensible. Lorsqu'un gaz dont on souhaite mesurer la quantité arrive en contact avec la couche sensible, des réactions d'oxydo-réductions sont entraînées, faisant varier les caractéristiques de la couche sensible, notamment sa résistance qui est mesurée afin de détecter une telle variation et ainsi de déterminer la quantité du gaz. La quantité de gaz permet de quantifier un gaz mais également de détecter sa présence.

En pratique, un tel capteur n'est pas performant pour détecter une pluralité de gaz. Une solution consiste à multiplier les capteurs de gaz hétérogènes afin d'améliorer la détection. Néanmoins, une telle solution présente l'inconvénient d'augmenter le coût de détection de manière significative.

Une autre solution consiste à faire varier des paramètres d'un unique capteur de gaz afin de le rendre sensible à plusieurs gaz de natures différentes. Aussi, il a été proposé d'alimenter la couche chauffante par une tension variant par paliers. Ceci permet de faire varier la température de la couche sensible afin qu'elle mesure différents gaz. Cependant, le nombre de mesure étant limité, il n'est pas possible de discriminer un nombre important de gaz. De plus, le capteur étant alimenté par une tension continue, sa durée de vie est limitée.

Par ailleurs, il a été proposé d'appliquer un signal carré avec modulation de type PWM afin de faire varier la tension d'alimentation de la couche chauffante. Bien que cette solution permette de réaliser un nombre plus important de mesure, les mesures réalisées sont sensibles aux variations de la température extérieure et de l'humidité. Aussi, la répétabilité des mesures n'est pas garantie lorsque les conditions extérieures varient. De plus, la commande par un signal PWM dépend des caractéristiques intrinsèques du capteur qui varie d'un capteur à l'autre, ce qui pénalise encore la répétabilité des mesures.

Il est également connu par l'article publié par Kohler H et al sous l'intitulé « New applications of tin oryde gas sensors - I. Molecular identification by cyclic variation of the working température and numerical analysis of the signais » et par la demande de brevet correspondante DE19639072A1, un procédé et un système d'identification moléculaire par variation cyclique de la température d'un capteur semi-conducteur sur une période. Lors d'une phase de calibration, le capteur est placé successivement dans des milieux connus comprenant des solvants connus à des concentrations connues et mesure pour chacun un profil de conductance sur toute la période. Lors d'une phase d'identification, le capteur est placé dans un milieu inconnu comprenant un ou plusieurs des solvants connus de la phase de calibration et mesure un profil de conductance sur toute la période. La comparaison du profil de conductance du milieu inconnu complet avec les profils de conductance des milieux connus complets permet de déterminer une estimation de la concentration relative de chaque substance connue dans le milieu inconnu.

On connaît par ailleurs par la demande de brevet US5889198A1 un procédé et un système de détection de méthane au moyen d'un capteur semi-conducteur à oxyde de gallium. Le procédé consiste à mesurer une première conductance à une première température puis une deuxième conductance à une deuxième température différente de la première température, obtenue en chauffant le capteur avec une rampe de tension linéaire. La deuxième conductance combinée au ratio de la deuxième conductance sur la première conductance permet de déterminer la concentration en méthane associée par comparaison avec une table de données.

De manière incidente, on connaît par la demande de brevet WO2006057550A1 un procédé et un système de détection de polluants dans l'air, notamment au niveau d'un système de ventilation de véhicule, permettant de stopper la circulation d'air venant de l'extérieur lorsque le niveau de pollution extérieur est trop important. Le système de détection comprend un capteur électrochimique comprenant une couche sensible comprenant des électrodes et un élément chauffant la couche sensible suivant un profil de température comprenant une rampe croissante puis décroissante. Le système de détection est d'abord placé dans un ou plusieurs environnements de calibration comprenant des gaz connus en une concentration connue afin de mesurer et stocker des réponses au profil de température. Le système de détection est ensuite placé dans un environnement comprenant des gaz connus en une concentration inconnue. La réponse au profil de température est comparée aux réponses stockées pour déterminer la concentration de ces gaz connus.

Il existe donc un besoin pour un procédé de commande d'un capteur permettant de résoudre au moins certains de ces inconvénients.

## PRESENTATION GENERALE DE L'INVENTION

A cet effet, l'invention concerne un procédé de détection d'au moins une quantité de gaz d'au moins un gaz prédéterminé à partir d'un capteur de mesure d'une pluralité de gaz, ledit capteur comprenant une couche sensible configurée pour mesurer la pluralité de gaz ayant une impédance Zs et une couche chauffante sur laquelle est montée la couche sensible, ladite couche chauffante étant configurée pour être alimentée en énergie électrique afin de faire varier la température de la couche sensible, ledit procédé comprenant :

- une étape d'alimentation de la couche chauffante par au moins une rampe de tension définissant une évolution linéaire de la tension d'alimentation entre une valeur de tension basse et une valeur de tension haute afin de modifier la température de la couche sensible pendant une période de variation,

- une étape de mesure de variations de l'impédance Zs de la couche sensible à plusieurs températures de la couche sensible pendant la période de variation de manière à détecter une pluralité de quantité de gaz

- une étape de comparaison, à une base de données, d'au moins une variation de l'impédance Zs de la couche sensible mesurée à une température de la couche sensible donnée, afin d'associer la quantité de gaz mesurée à un gaz prédéterminé.

L'invention concerne plus précisément un procédé de détection d'au moins une quantité de gaz d'au moins un gaz prédéterminé à partir d'un capteur de mesure d'une pluralité de gaz, ledit capteur comprenant une couche sensible configurée pour mesurer la pluralité de gaz ayant une impédance Zs et une couche chauffante sur laquelle est montée la couche sensible, ladite couche chauffante étant configurée pour être alimentée en énergie électrique afin de faire varier la température de la couche sensible, ledit procédé comprenant :

- une étape d'alimentation de la couche chauffante par au moins une première rampe croissante de tension définissant une évolution linéaire de la tension d'alimentation puis par une deuxième rampe décroissante de tension définissant une évolution linéaire de la tension d'alimentation entre une valeur de tension basse et une valeur de tension haute afin de modifier la température de la couche sensible pendant une période de variation selon une rampe de température comprenant une partie croissante et une partie décroissante, la rampe de température comprenant une pluralité de plages de température ΔT correspondant chacune à un gaz à mesurer,

- une étape de mesure :

- d'une première variation de l'impédance Zs de la couche sensible à une plage de température donnée ΔT de la couche sensible lors de la première rampe croissante et

- d'une deuxième variation de l'impédance Zs de la couche sensible mesurée à la même plage de température donnée ΔT de la couche sensible lors de la deuxième rampe décroissante, la plage de température ΔT correspondant à un gaz donné à mesurer ;

- une étape de comparaison, à une base de données, de la première variation de l'impédance Zs et de la deuxième variation de l'impédance Zs afin de déterminer la quantité de gaz associée audit gaz donné.

Grâce au procédé selon l'invention, il est possible de mesurer différents gaz avec le capteur de mesure grâce à la modification de la température de la couche sensible qui la rend sensible à des gaz différents. De plus, grâce à l'alimentation de la couche chauffante par une rampe, la température de la couche sensible évolue linéairement et de manière précise. La détection d'une variation associée à une température de la couche sensible permet d'associer de manière précise et fiable la quantité de gaz mesurée à un gaz prédéterminé. La quantité de gaz permet de quantifier un gaz mais également de détecter sa présence. Autrement dit, le procédé selon l'invention permet de déterminer la présence d'un gaz uniquement à partir de la détection d'une variation d'impédance lors de la première rampe croissante et lors de la deuxième rampe décroissante. La plage de température ΔT à laquelle est mesurée la variation d'impédance est réduite, identique pour la première rampe croissante et la deuxième rampe décroissante et caractéristique d'un gaz donné. La plage de température ΔT permet ainsi de déterminer la nature du gaz et l'amplitude de la variation d'impédance permet de déterminer la quantité dudit gaz. Ainsi, lorsqu'un gaz induit une variation d'impédance Zs différente pour une rampe croissante ou une rampe décroissante, on peut déterminer sa quantité de manière plus précise par analyse des des variations d'impédance.

De préférence, pendant la période de variation, la couche chauffante étant alimentée par une première rampe croissante puis par une deuxième rampe décroissante, une première variation de l'impédance Zs de la couche sensible, mesurée à une température de la couche sensible donnée lors de la première rampe croissante, et une deuxième variation de l'impédance Zs de la couche sensible, mesurée à une température de la couche sensible donnée lors de la deuxième rampe décroissante, sont comparées à la base de données afin d'associer les quantité de gaz mesurées à un gaz prédéterminé.

Ainsi, plusieurs variations de l'impédance Zs de la couche sensible peuvent être mesurées deux fois à une même température : lors de la première rampe et lors de la deuxième rampe. De manière avantageuse, le comportement de la couche sensible peut être différent selon que la température augmente ou diminue, ce qui permet d'améliorer l'identification du gaz mesuré. La discrimination des gaz est améliorée

De manière préférée, le procédé comprend une étape de détermination de la température Ts de la couche sensible à partir de la résistance thermique Rth déterminée, de la puissance P générée par la couche chauffante (12) et d'une mesure de la température ambiante Ta selon la formule suivante :

$$T_S = P * R_{th} + T_A$$

De manière préférée, la température ambiante Ta est mesurée par un capteur de température extérieur au capteur de gaz.

De préférence, le procédé comprenant une étape préliminaire de détermination de la résistance thermique Rth de la couche chauffante par mise en contact du capteur de gaz avec un gaz étalon prédéterminé. Ainsi, la détermination de la température de la couche sensible est optimale étant donné que la résistance thermique Rth est déterminé de manière individuelle pour chaque capteur de gaz.

De manière préférée, l'amplitude de variation thermique est d'au moins 200°C, de préférence, d'au moins 300°C. De préférence, la plage de variation de température est comprise entre 100°C et la température maximale acceptée par le capteur, par exemple de l'ordre de 500°C. Une telle plage de température permet d'atteindre les températures où des réactions d'oxydo-réductions apparaissent.

De préférence, la fréquence de variation thermique est $F_{th}$ est comprise entre 0,1 Hz et 1 Hz soit une plage de variation de 1 seconde à 10 secondes.

L'invention concerne également un ensemble d'un capteur de mesure d'une pluralité de gaz et d'un calculateur de commande dudit capteur, ledit capteur comprenant une couche sensible configurée pour mesurer ledit au moins un gaz ayant une impédance Zs et une couche chauffante sur laquelle est montée la couche sensible ladite couche chauffante étant configuré pour être alimentée en énergie électrique afin de faire varier la température de la couche sensible, ledit calculateur étant configuré pour mettre en oeuvre le procédé tel que présenté précédemment.

De préférence, le calculateur comprend une zone mémoire comprenant la base de données pour la mise en oeuvre du procédé tel que présenté précédemment.

## PRESENTATION DES FIGURES

L'invention sera mieux comprise à la lecture de la description qui va suivre, donnée uniquement à titre d'exemple, et se référant aux dessins annexés sur lesquels :

Fig. 1 est une représentation schématique d'une forme de réalisation d'un capteur de mesure de gaz selon l'invention,
Fig. 2 est une représentation schématique d'une courbe d'évolution de la température du capteur de la figure 1 au cours du temps,
Fig. 3 est une représentation schématique d'une courbe d'évolution de l'impédance du capteur de la figure 1 lorsque la température varie au cours du temps, et
Fig. 4 est un exemple de mise en oeuvre d'un procédé de commande d'un capteur de mesure selon l'invention.

Il faut noter que les figures exposent l'invention de manière détaillée pour mettre en oeuvre l'invention, lesdites figures pouvant bien entendu servir à mieux définir l'invention le cas échéant.

## DESCRIPTION DETAILLEE D'UNE FORME DE REALISATION DE L'INVENTION

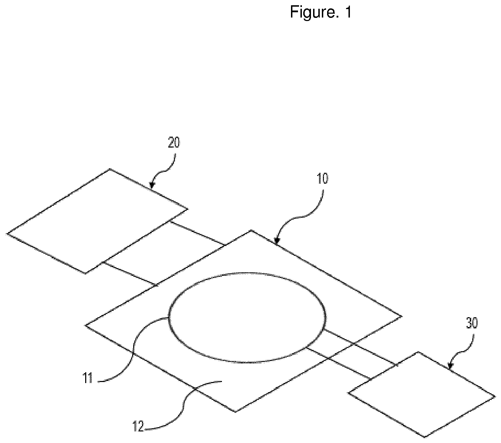

En référence à la figure 1, il est représenté de manière schématique un capteur de mesure 10 d'un gaz selon l'invention. Le capteur de mesure 10 est alimenté en énergie par une source d'alimentation 20 et un calculateur 30 est configuré pour déterminer la quantité de gaz mesurée par le capteur 10.

Le capteur de mesure 10 se présente sous la forme d'un capteur oxydo-métallique, également désigné MOX. Un tel capteur 10 comprend une couche sensible 11 à au moins un gaz et une couche chauffante 12 sur laquelle est montée ladite couche sensible 11.

La couche sensible 11 est configurée pour réagir avec un gaz afin d'en détecter la présence au niveau du capteur de mesure 10.

La couche sensible 11 est en contact avec l'air et est adaptée pour absorber des molécules du gaz à mesurer. L'absorption de ces molécules fait varier la conductivité électrique à travers la couche sensible 11, et ainsi l'impédance ZS aux bornes de la couche sensible 11, grâce à une réaction d'oxydo-réduction, ce qui permet de déterminer la quantité dudit gaz présent dans l'air en contact avec la couche sensible 11.

La couche sensible 11 comprend un oxyde permettant la réaction d'oxydo-réduction avec le gaz, par exemple, des molécules de dioxyde d'étain (SnO2), de dioxyde de titanium (TiO2), de trioxyde de tungstène (WO3) et/ou de l'oxyde de Niobium (Ne2O5) avec des dopages d'éléments de Platine (Pt), d'or (Au), de Germanium (Ge) et/ou de Palladium (Pd). Les éléments chimiques qui sont mélangés avec des molécules permettent de doper ces dernières afin d'optimiser leur fonction d'absorption de molécules du gaz à mesurer.

Lorsque des molécules du gaz à mesurer sont absorbées par la couche sensible 11, l'impédance ZS à ses bornes varie, ce qui permet de déterminer la présence du gaz à mesurer ainsi que sa quantité. Autrement dit, la quantité de gaz présent dans l'air est mesurée à partir de la variation de l'impédance de la couche sensible 11. Pour mesurer la variation de l'impédance, la couche sensible 11 est alimentée en énergie électrique par le calculateur 30 comme cela sera décrit par la suite. Dans ce but, la couche sensible 11 est reliée électriquement au calculateur 30.

La couche chauffante 12 est commandée afin de modifier la température de la couche sensible 11.

Une telle modification de sa température permet à la couche sensible 11 d'absorber différents gaz selon la température de la couche sensible 11. Autrement dit, chaque température de la couche sensible 11 permet de mesurer la quantité d'un gaz. Ainsi, le capteur de mesure 10 est configuré pour mesurer différents gaz en modifiant la température de la couche sensible 11. La couche chauffante 12 est alimentée en énergie électrique afin d'en commander la température comme cela sera décrit par la suite. Dans ce but, la couche chauffante 12 est reliée électriquement à la source d'alimentation 20.

Grâce à la couche chauffante 12, une seule couche sensible 11 permet de mesurer différents gaz. Ceci permet d'utiliser un unique capteur de mesure 10 pour mesurer la quantité de plusieurs gaz, ce qui limite la consommation en énergie électrique et le coût de telles mesures.

La couche chauffante 12 comprend dans cet exemple un substrat, tel que de la céramique ou du silicium, et des éléments, tels que de l'or, adaptés pour dégager de la chaleur lorsqu'ils sont traversés par un courant électrique.

Selon l'invention, la température TS de la couche sensible 11 est commandée par la couche chauffante 12 afin de permettre des mesures précises de la couche sensible 11. Dans ce but, la température TS de la couche sensible 11 est déterminée à partir de l'équation suivante :

$$T_S = P * R_{th} + T_A$$

dans laquelle P est la puissance générée par la couche chauffante 12, Rth est la résistance thermique de la couche chauffante 12 à la couche sensible 11 et TA est la température ambiante.

Autrement dit, la température TS de la couche sensible 11 dépend de la température ambiante TA et de la puissance P générée par la couche chauffante 12. L'utilisation de la résistance thermique Rth permet de prendre en compte les pertes de transfert de chaleur entre la couche chauffante 12 et la couche sensible 11.

La température TS de la couche sensible 11 est ainsi déterminée de manière précise à partir de la puissance P générée par la couche chauffante 12 à partir de l'énergie électrique fournie par la source d'alimentation 20. La plage de température sur laquelle la couche sensible 11 mesure un gaz déterminé étant réduite, la détermination précise de la température de la couche sensible 11 permet de déterminer de manière précise le gaz qui est mesuré par la couche sensible 11.

La température ambiante TA est mesurée par un capteur de température, ce qui permet d'obtenir une mesure précise. Un tel capteur de température peut être extérieur au capteur de mesure 10 ou bien compris dans le capteur de mesure 10.

La résistance thermique Rth de la couche chauffante 12 correspond aux pertes de chaleurs entre la couche chauffante 12 et la couche sensible 11. La résistance thermique Rth est indépendante du gaz auquel est soumis le capteur de mesure 10. Aussi, elle est déterminée préalablement aux mesures effectuées par le capteur de mesure 10. Elle peut notamment être déterminée lors de la conception du capteur de mesure 10. Si la résistance thermique Rth varie d'un capteur de mesure 10 à l'autre, elle peut être déterminée pour chaque capteur de mesure 10 après sa fabrication. Dans ce but, chaque capteur de mesure 10 est mis en présence d'un gaz étalon. Un tel gaz étalon est un gaz pour lequel le comportement de la couche sensible 11 est connu. La couche chauffante 12 est alors soumise à une courbe théorique de puissance P de manière à ce que la couche sensible 11 détecte le gaz étalon. La puissance réelle à laquelle la couche sensible 11 détecte le gaz étalon étant connue, la résistante thermique Rth est déterminée à partir du décalage entre la puissance de la courbe théorique à laquelle la couche sensible 1 a détectée le gaz étalon et la puissance réelle. Suite à cette étape de calibrage, la résistance thermique Rth est stockée dans le calculateur 30 afin de permettre de contrôler la couche sensible 11 de manière optimale.

La source d'alimentation 20 est configurée pour alimenter la couche chauffante 12 en énergie électrique afin que cette dernière génère de la chaleur. Comme illustré sur la figure 1, la source d'alimentation 20 est reliée électriquement à la couche chauffante 12.

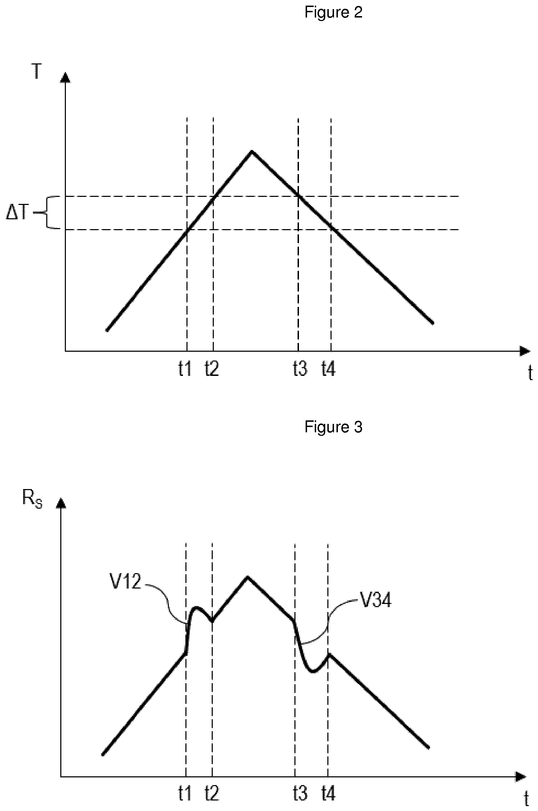

Selon l'invention, la source d'alimentation 20 est configurée pour alimenter la couche chauffante 12 par une rampe de tension (non représentée) comprenant une partie croissante et une partie décroissante. La tension d'une telle rampe évolue ainsi linéairement et lentement afin que la température de la couche sensible 11 évolue également linéairement et lentement en formant une rampe comme illustrée sur la figure 2. La rampe de température permet ainsi d'effectuer une pluralité de mesure pour détecter différents gaz. Pour ce faire, la rampe de température comprend une pluralité de plage de température ΔT correspondant chacune à un gaz à mesurer. Par soucis de clarté, une unique plage de température ΔT a été illustrée sur la figure 2 correspondant à la mesure d'un unique gaz. Cependant, il va de soi que la rampe de température comprend autant de plage de température ΔT que de gaz à mesurer.

Comme illustrée sur la figure 2, la rampe de température comprenant une partie croissante et une partie décroissante, chaque plage de température ΔT est comprise dans la partie croissante et dans la partie décroissante. Autrement dit, la rampe de température passe par une plage de température ΔT lors de l'augmentation de la température (entre les instants t1 et t2) et lors de la diminution de la température (entre les instants t3 et t4). Ceci permet de détecter un même gaz plusieurs fois et à différents instants de la rampe de température. Ceci permet également d'identifier aisément les gaz pour lesquels le comportement de la couche sensible 11 diffère selon que la température augmente ou diminue comme cela sera décrit par la suite. La présence d'une montée et d'une descente forme ainsi un critère de discrimination des gaz comme cela sera présenté par la suite.

La source d'alimentation 20 est commandée par un calculateur, qui peut être le calculateur 30 de commande.

Le calculateur 30 de commande est configuré pour déterminer la quantité d'une pluralité de gaz dans l'air.

Comme illustré sur la figure 1, le calculateur 30 est relié électriquement à la couche sensible 11 afin de mesurer l'impédance de la couche sensible 11 et d'en déterminer la présence d'un gaz. Dans ce but, le calculateur 30 alimente la couche sensible 11 en énergie électrique. De préférence, le calculateur 30 est configuré pour émettre un signal de tension continu, périodique ou pseudo-périodique à la couche sensible 11.

Comme illustré sur la figure 3, l'impédance Zs de la couche sensible 11 évolue avec la température. L'impédance Zs suit une évolution linéaire due à l'évolution linéaire de la température. En présence d'un gaz déterminé, l'impédance ZS de la couche sensible 11 varie de manière non linéaire, formant notamment un pic comme illustré sur la figure 3, sur une plage de température ΔT associée au gaz déterminé. Une telle variation non linéaire permet ainsi de détecter un gaz et d'en mesurer la quantité. Avantageusement, la variation de l'impédance ZS sur la plage de température ΔT peut être, pour certains gaz, différente selon que la température évolue de manière croissante ou de manière décroissante, autrement dit selon que l'on soit dans la partie croissante ou décroissante de la rampe de température. Ceci permet d'augmenter la fiabilité de la détection d'un gaz.

Le calculateur 30 comprend avantageusement une zone mémoire comprenant une base de données comprenant les différentes formes de variation de l'impédance ZS de la couche sensible 11 selon le gaz mesuré associées à la température Ts de la couche sensible 11. Ceci permet d'identifier le gaz détecté par comparaison entre la variation de l'impédance ZS mesurée et les variations enregistrées. De manière préférée, comme cela sera présenté par la suite, la base de données associe un gaz prédéterminé à une plage de température Ts de la couche sensible et à une ou plusieurs variations de l'impédance Zs, en particulier, à une variation montante et à une variation descendante. La base de données peut être obtenue de manière empirique ou à partir de données calculées.

L'impédance ZS est mesurée à partir de la détermination de la valeur de la tension U aux bornes de la couche sensible 11. Cette tension U peut notamment être déterminée à l'aide d'un pont diviseur de tension dans le cas d'un signal de tension continue généré par le calculateur 30.

Dans le cas d'un signal de tension périodique ou pseudo-périodique, notamment de type chirp, la couche sensible 11 peut alors être assimilée à un circuit RLC ce qui permet de calculer la tension U à partir de l'équation :

$$U = U_C + U_L + U_R \qquad (1)$$

Dans laquelle UC est la tension aux bornes de la capacité C du circuit RLC, UL est la tension aux bornes de la bobine L du circuit RLC et UR est la tension aux bornes de la résistance R du circuit RLC.

L'équation (1) peut également s'écrire sous la forme :

$$U = -\frac{j}{C\omega} I + jL\omega I + RI \quad (2)$$

Avec $\omega = 2\pi f$ où f est la fréquence du signal de tension.

Lorsque la fréquence f du signal est égal à la fréquence de résonnance du système RLC, l'équation (2) devient :

$$U = RI$$

Aussi, en utilisant la fréquence de résonnance, il est aisé de déterminer la résistance R du système RLC qui est égale à l'impédance ZS de la couche sensible 11. La fréquence de résonnance du système RLC peut être déterminée en balayant toutes les fréquences et en déterminant la fréquence pour laquelle la valeur du courant I est la plus élevée. De manière alternative, la fréquence de résonnance du système RLC peut être déterminée en balayant toutes les fréquences et en déterminant la fréquence pour laquelle le déphase entre I et U et nul.

L'utilisation d'un tel système RLC est également avantageuse car elle permet de mesurer, outre des variations de l'impédance ZS de la couche sensible 11, des variations de type capacitives, autrement dit de la capacité C du système RLC, et selfiques, autrement dit de la bobine L. Ces différentes variations peuvent alors être utilisées pour déterminer le gaz détecté, la zone mémoire du calculateur 30 comprenant alors les différentes formes de ces variations selon le gaz mesuré et selon la température de la couche sensible.

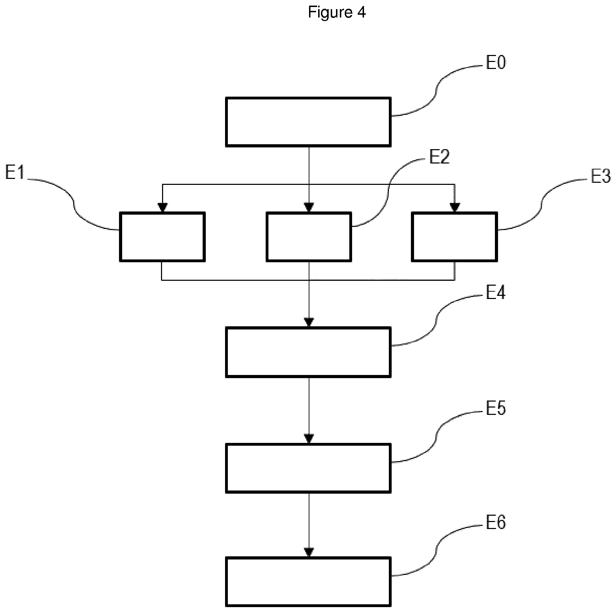

Il va maintenant être décrit une forme de mise en oeuvre du procédé de commande d'un capteur de mesure 10 selon l'invention en référence aux figures 2 à 4.

Lors d'une étape préliminaire E0, qui peut notamment être réalisée en sortie d'usine, la résistance thermique Rth de la couche chauffante 12 est déterminée. Dans ce but, le capteur de mesure 10 est mis en présence d'un gaz étalon pour lequel le comportement du capteur de mesure 10 est connu.

Lors de l'utilisation du capteur de mesure 10, une rampe de tension est appliquée, lors d'une étape E1, à la couche chauffante 12 afin de faire varier la température de la couche sensible 11.

La température TS de la couche sensible 11 est déterminée en continu, lors d'une étape E2, à partir de l'équation :

$$T_S = P * R_{th} + T_A$$

dans laquelle P est la puissance générée par la couche chauffante 12, Rth est la résistance thermique de la couche chauffante 12 et TA est la température ambiante. La température TS suit alors une rampe comme illustré sur la figure 2.

De manière connue, la puissance P générée par la couche chauffante 12 est directement fonction de la tension appliquée à la couche chauffante 12 et peut être déduite de manière pratique.

Le calculateur 30 mesure en outre, dans une étape E3, l'impédance ZS de la couche sensible 11 de manière à générer une courbe d'évolution de l'impédance ZS comme illustré sur la figure 3.

Dans une étape E4, en référence à la figure 3, le calculateur 30 détecte deux variations non linéaires V12, V34 de l'impédance Zs sur la courbe d'évolution. Le calculateur 30 détermine alors l'intervalle de temps (entre les instants t1 et t2, d'une part, et entre les instants t3 et t4, d'autre part comme illustré sur la figure 3) pendant lesquels les variations V12, V34 ont été détectées. Dans cet exemple, une variation convexe V12 est détectée entre les instants t1 et t2 tandis qu'une variation concave V34 est détectée entre les instants t3 et t4.

Le calculateur 30 détermine alors la plage de température ΔT correspondant aux intervalles de temps déterminés à partir de la rampe d'évolution de la température.

Le calculateur 30 compare alors dans une étape E5 les variations V12, V34 aux variations stockées dans la zone mémoire du calculateur 30 par correspondance de forme de variation et de plage de température ΔT.

Enfin, le calculateur 30 identifie le gaz détecté à partir des similitudes dans la comparaison avec les variations enregistrées. Autrement dit, le gaz est identifié comme étant celui correspondant à la variation enregistrée dont la forme est la plus proche de la forme de la variation détectée. La mesure de gaz peut ainsi être associée à un gaz prédéterminé. L'utilisation d'une variation à la monté et à la descente permet d'améliorer les performances de discrimination.

Grâce à la variation de température, il est ainsi possible de détecter différents gaz durant cette variation grâce à la détection de variations non linéaires de l'impédance Zs de la couche sensible 11. Chaque variation détectée est séparée des autres par plage de température afin de les comparer aux variations présentes dans la base de données et ainsi de détecter la nature des différents gaz mesurés. Autrement dit, la commande judicieuse d'un unique capteur de gaz permet de mesurer la quantité d'une pluralité de gaz différents. Un tel capteur est performant tout en restant économique.

**Revendications**

1. Procédé de détection d'au moins une quantité de gaz d'au moins un gaz prédéterminé à partir d'un capteur (10) de mesure d'une pluralité de gaz, ledit capteur (10) comprenant une couche sensible (11) configurée pour mesurer la pluralité de gaz ayant une impédance Zs et une couche chauffante (12) sur laquelle est montée la couche sensible (11), ladite couche chauffante (12) étant configurée pour être alimentée en énergie électrique afin de faire varier la température de la couche sensible (11), ledit procédé comprenant :

   - une étape d'alimentation (E1) de la couche chauffante (12) par au moins une première rampe croissante de tension définissant une évolution linéaire de la tension d'alimentation puis par une deuxième rampe décroissante de tension définissant une évolution linéaire de la tension d'alimentation entre une valeur de tension basse et une valeur de tension haute afin de modifier la température de la couche sensible (11) pendant une période de variation selon une rampe de température comprenant une partie croissante et une partie décroissante,
   - une étape (E2) de détermination de la température Ts de la couche sensible (11) à partir d'une résistance thermique Rth déterminée, de la puissance P générée par la couche chauffante (12) et d'une mesure de la température ambiante Ta selon la formule suivante : $T_S = P * R_{th} + T_A$
   - une étape de mesure (E3) de variations de l'impédance Zs de la couche sensible (11) à plusieurs températures de la couche sensible (11) pendant la période de variation de manière à détecter une pluralité de quantité de gaz,
   - une étape de détection (E4) :

      - d'une première variation non linéaire de l'impédance Zs de la couche sensible (11) à une plage de température donnée ΔT de la couche sensible (11) lors de la première rampe croissante et
- d'une deuxième variation non linéaire de l'impédance Zs de la couche sensible (11) mesurée à la même plage de température donnée ΔT de la couche sensible (11) lors de la deuxième rampe décroissante,

- une étape de comparaison de la première variation de l'impédance Zs et de la deuxième variation de l'impédance Zs à une base de données associant un gaz prédéterminé à une plage de température de la couche sensible, à une variation montante de l'impédance Zs et à une variation descendante de l'impédance Zs, les plages de température correspondant chacune à un gaz à mesurer,
- l'étape de comparaison étant mise en oeuvre en comparant la première variation et deuxième variation non linéaire de l'impédance Zs mesurées avec les variations montante et descendante stockées dans la base de données par correspondance de forme de variation et de plage de température, afin d'associer la quantité de gaz mesurée à un gaz prédéterminé.

2. Procédé selon la revendication 1, dans lequel le procédé comprenant une étape préliminaire (E0) de détermination de la résistance thermique Rth de la couche chauffante (12) par mise en contact du capteur de gaz (10) avec un gaz étalon prédéterminé.

3. Procédé selon l'une des revendications 1 et 2, dans lequel l'amplitude de variation thermique est d'au moins 400°C.

4. Procédé selon l'une des revendications 1 à 3, dans lequel la fréquence de variation thermique est comprise entre 0,1Hz et 1 Hz.

5. Ensemble d'un capteur (10) de mesure d'une pluralité de gaz et d'un calculateur (30) de commande dudit capteur (10), ledit capteur (10) comprenant une couche sensible (11) configurée pour mesurer ledit au moins un gaz ayant une impédance Zs et une couche chauffante (12) sur laquelle est montée la couche sensible (11), ladite couche chauffante (12) étant configuré pour être alimentée en énergie électrique afin de faire varier la température de la couche sensible (11), ledit calculateur (30) étant configuré pour mettre en oeuvre le procédé selon l'une des revendications 1 à 4.

6. Ensemble selon la revendication précédente, dans lequel le calculateur (30) comprenant une zone mémoire comprenant la base de données pour la mise en oeuvre du procédé selon l'une des revendications 1 à 4.

**Patentansprüche**

1. Verfahren zur Detektion mindestens einer Gasmenge mindestens eines vorbestimmten Gases mit einem Sensor (10) zum Messen einer Vielzahl von Gasen, wobei der Sensor (10) eine empfindliche Schicht (11), die zum Messen der Vielzahl von Gasen ausgelegt ist, mit einer Impedanz Zs und eine Heizschicht (12) umfasst, auf der die empfindliche Schicht (11) angebracht ist, wobei die Heizschicht (12) ausgelegt ist, um mit elektrischer Energie versorgt zu werden, um die Temperatur der empfindlichen Schicht (11) zu ändern, wobei das Verfahren umfasst:

- einen Schritt des Versorgens (E1) der Heizschicht (12) mit mindestens einer ersten ansteigenden Spannungsrampe, die einen linearen Verlauf der Versorgungsspannung definiert, dann mit einer zweiten abfallenden Spannungsrampe, die einen linearen Verlauf der Versorgungsspannung zwischen einem niedrigen Spannungswert und einem hohen Spannungswert definiert, um die Temperatur der empfindlichen Schicht (11) während einer Änderungsperiode gemäß einer Temperaturrampe, die einen ansteigenden Teil und einen abfallenden Teil umfasst, zu ändern,
- einen Schritt (E2) des Bestimmens der Temperatur Ts der empfindlichen Schicht (11) ausgehend von dem bestimmten Wärmewiderstand Rth, der von der Heizschicht (12) erzeugten Leistung P und einer Messung der Umgebungstemperatur Ta gemäß der folgenden Formel: $Ts = P * R_{th} + T_A$
- einen Schritt (E3) des Messens von Änderungen der Impedanz Zs der empfindlichen Schicht (11) bei mehreren Temperaturen der empfindlichen Schicht (11) während der Änderungsperiode, um eine Gasmengenvielzahl zu ermitteln,
- einen Detektionsschritt (E4):

- einer ersten nichtlinearen Änderung der Impedanz Zs der empfindlichen Schicht (11) in einem gegebenen Temperaturbereich ΔT der empfindlichen Schicht (11) während der ersten ansteigenden Rampe und
- einer zweiten nichtlinearen Änderung der Impedanz Zs der empfindlichen Schicht (11) im gleichen Temperaturbereich ΔT der empfindlichen Schicht (11) während der zweiten abfallenden Rampe,

- einen Schritt des Vergleichens der ersten Änderung der Impedanz Zs und der zweiten Änderung der Impedanz Zs mit einer Datenbank, die ein vorbestimmtes Gas einem Temperaturbereich der empfindlichen Schicht mit einer ansteigenden Änderung der Impedanz Zs und mit einer abfallenden Änderung der Impedanz Zs zuordnet, wobei die Temperaturbereiche jeweils einem zu messenden Gas entsprechen,
- wobei der Vergleichsschritt durchgeführt wird, indem die nichtlineare gemessene erste Änderung und gemessene zweite Änderung der Impedanz Zs mit den in der Datenbank gespeicherten ansteigenden und abfallenden Änderungen durch Übereinstimmung der Änderungsform und des Temperaturbereichs verglichen wird, um die gemessene Gasmenge einem vorbestimmten Gas zuzuordnen.

2. Verfahren nach Anspruch 1, wobei das Verfahren einen einleitenden Schritt (E0) des Bestimmens des Wärmewiderstands Rth der Heizschicht (12) durch Inkontaktbringen des Gassensors (10) mit einem vorbestimmten Eichgas umfasst.

3. Verfahren nach einem der Ansprüche 1 und 2, wobei die Amplitude der thermischen Änderung mindestens 400 °C beträgt.

4. Verfahren nach einem der Ansprüche 1 bis 3, wobei die Frequenz der thermischen Änderung zwischen 0,1 Hz und 1 Hz liegt.

5. Anordnung aus einem Sensor (10) zum Messung einer Vielzahl von Gasen und einem Rechner (30) zur Steuerung des Sensors (10), wobei der Sensor (10) eine empfindliche Schicht (11), die zum Messen der Vielzahl von Gasen ausgelegt ist, mit einer Impedanz Zs und eine Heizschicht (12) umfasst, auf der die empfindliche Schicht (11) angebracht ist, wobei die Heizschicht (12) ausgelegt ist, um mit elektrischer Energie versorgt zu werden, um die Temperatur der empfindlichen Schicht (11) zu ändern, wobei der Rechner (30) ausgelegt ist, um das Verfahren nach einem der Ansprüche 1 bis 4 auszuführen.

6. Anordnung nach vorhergehendem Anspruch, wobei der Rechner (30) einen Speicherbereich umfasst, der die Datenbank zur Durchführung des Verfahrens nach einem der Ansprüche 1 bis 4 umfasst.

**Claims**

1. A method for detecting at least one gas quantity of at least one predetermined gas from a sensor (10) for measuring a plurality of gases, said sensor (10) comprising a sensitive layer (11) configured to measure the plurality of gases having an impedance Zs and a heating layer (12) on which the sensitive layer (11) is mounted, said heating layer (12) being configured to be supplied with electrical energy in order to vary the temperature of the sensitive layer (11), said method comprising :

- a step (E1) of supplying the heating layer (12) with at least a first increasing voltage ramp defining a linear change of the supply voltage, then with a second decreasing voltage ramp defining a linear change of the supply voltage between a low voltage value and a high voltage value, in order to modify the temperature of the sensitive layer (11) during a variation period according to a temperature ramp comprising an increasing part and a decreasing part,
- a step (E2) for determining the temperature Ts of the sensitive layer (11) from a determined thermal resistance Rth, the power P generated by the heating layer (12) and a measurement of the ambient temperature Ta according to the following formula: $Ts = P * R_{th} + T_A$
- a step (E3) of measuring variations in the impedance Zs of the sensitive layer (11) at several temperatures of the sensitive layer (11) during the variation period, so as to detect a plurality of gas quantities,
- a detection step (E4) for detecting:

  - a first non-linear variation of the impedance Zs of the sensitive layer (11) at a given temperature range $\Delta T$ of the sensitive layer (11) during the first ramp-up and
  - a second non-linear variation of the impedance Zs of the sensitive layer (11) measured at the same given temperature range $\Delta T$ of the sensitive layer (11) during the second decreasing ramp,

- a step of comparing the first variation of the impedance Zs and the second variation of the impedance Zs with a database associating a predetermined gas with a temperature range of the sensitive layer, with a rising variation in impedance Zs and with a falling variation of the impedance Zs, the temperature ranges each corresponding to a gas to be measured,
- the step of comparing being implemented by comparing the first variation and second non-linear variation of the impedance Zs measured with the upward and downward variations stored in the database by correspondence of variation form and temperature range, in order to associate the quantity of gas measured with a predetermined gas.

2. The method according to claim 1, wherein the method comprises a preliminary step (E0) of determining the thermal resistance Rth of the heating layer (12) by bringing the gas sensor (10) into contact with a predetermined standard gas.

3.  The method according to one of claims 1 and 2, wherein the amplitude of thermal variation is at least 400°C.

4.  The method according to one of claims 1 to 3, wherein the frequency of thermal variation is between 0.1 Hz and 1 Hz.

5.  An assembly of a sensor (10) for measuring a plurality of gases and a computer (30) for controlling said sensor (10), said sensor (10) comprising a sensitive layer (11) configured to measure said at least one gas having an impedance Zs and a heating layer (12) on which the sensitive layer (11) is mounted, said heating layer (12) being configured to be supplied with electrical energy in order to vary the temperature of the sensitive layer (11), said computer (30) being configured to implement the method according to one of claims 1 to 4.

6.  The assembly according to the preceding claim, wherein the computer (30) comprises a memory area comprising the database for implementing the method according to one of claims 1 to 4.

Figure. 1

Figure 2

Figure 3

Figure 4

## RÉFÉRENCES CITÉES DANS LA DESCRIPTION

**Documents brevets cités dans la description**

- DE 19639072 A1
- US 5889198 A1
- WO 2006057550 A1